# EUROPEAN PATENT SPECIFICATION

(11) **EP 1 148 128 B1**
(45) Date of publication and mention of the grant of the patent: **06.09.2006**
(21) Application number: 01201037.7
(22) Date of filing: 20.03.2001
(51) Int. Cl.: C12N 15/12, C07K 14/47, C12Q 1/68, A61K 48/00, A61P 27/00, A01K 67/027

(54) **Gene locus involved in regulating hair pigmentation, vestibular function and fertility**
Genort, beteiligt an der Regulation der Haarpigmentierung, Vestibularfunktion und Fruchtbarkeit
Locus du gène involvé à reguler la pigmentation des cheveux, la fonction vestibulaire et la fertilité

(30) Priority: 20.03.2000 US 528928
(43) Date of publication of application: 24.10.2001
(73) Proprietor: UNIVERSITY OF HONG KONG, Hong Kong (CN)
(72) Inventor: Cheah, Kathryn S.E., Hong Kong (CN)
(74) Representative: Prins, Adrianus Willem

(56) References cited:
- US-A- 5 723 719
- MICHAUD EDWARD J ET AL: "The embryonic lethality of homozygous lethal yellow mice (A-y/A-y) is associated with the disruption of a novel RNA-binding protein." GENES & DEVELOPMENT, vol. 7, no. 7A, 1993, pages 1203-1213, XP002076781 ISSN: 0890-9369
- LANG R: "THE MAMMALIAN SPOT TEST AND ITS USE FOR TESTING OF MUTAGENIC AND CARCINOGENIC POTENTIAL EXPERIENCE WITH THE PESTICIDE CHLORDIMEFORM ITS PRINCIPAL METABOLITES AND THE DRUG LISURIDE HYDROGEN MALEATE" MUTATION RESEARCH, vol. 135, no. 3, 1984, pages 219-224, XP000926604 AMSTERDAM NL ISSN: 0027-5107
- DATABASE SWALL [Online] 1 November 1999 (1999-11-01) TAKADA, S. ET AL: "Mouse cDNA similar to 5' region of human EXML1 cDNA" retrieved from EMBL-EBI, accession no. Q9WTN9 Database accession no. Q9WTN9 XP002174568
- CHEAH KATHRYN S E ET AL: "Human COL2A1-directed SV40 T Antigen Expression in Transgenic and Chimeric Mice Results in Abnormal Skeletal Development." JOURNAL OF CELL BIOLOGY, vol. 128, no. 1-2, 1995, pages 223-237, XP000926594 NEW YORK US ISSN: 0021-9525
- DONG S. ET AL GENOMICS vol. 79, no. 6, June 2002, US, pages 777 - 784

## Description

### Background of the Invention

A mouse line bearing a recessive mutation, caused by insertion of a transgene, was created. These mice are characterized by a changed pigmentation of hair to give yellow coat color, circling behaviour and an inability to swim. It has been shown that the transgene has inserted into two sites on mouse chromosome 3. A search of the mouse genome database ascertained that the mutation is novel. The mutant locus has been named *yellow submarine* (*ysb*). DNA flanking the transgene insertion has been isolated and used to screen for the corresponding normal (unmutated) sequences. Genomic clones spanning 20kb of the unmutated *ysb* locus (+^{*ysb*}) have been isolated and mapping experiments indicate that transgene integration has caused a deletion and chromosomal inversion resulting in two transgene integration sites. Circular behaviour can reflect abnormality in the inner ear or be due to an abnormality in hindbrain development. Studies show abnormal structure of the inner ears of *ysb* mice and a stunted acoustic nerve. *Ysb* mice are therefore a novel mutant showing both abnormal regulation of pigmentation and inner ear dysfunction. Molecular genetics, bioinformatics, developmental biology, transgenic and physiological approaches are used to 1) identify and characterize the gene(s) at the wild-type *ysb* (+^{*ysb*}) locus; 2) determine the nature of the *ysb* mutation; 3) study the molecular and developmental bases underlying the defect(s) in *ysb* mice; and 4) charactersize the neurophysiological changes in balance and hearing in *ysb* mice. Approximately 1/1000 infants are affected by hearing defects at birth, two thirds of which have a genetic basis. *Ysb* mice are a valuable model to identify molecules involved in controlling balance. These studies provide fundamental information on the development of the inner ear and the mechanisms by which hearing and balance defects may arise. Identification and characterization of the *ysb* gene(s) and the molecular defect in *ysb* mice also yield new insight into the complex regulatory pathways controlling agouti coat color in mice.

This invention provides an isolated nucleic acid which defines a mutant yellow submarine locus, wherein the mutant yellow submarine locus is identical to a wildtype yellow submarine locus except for an integration of a pAA2 transgene into at least one region on a chromosome.

These isolated nucleic acid molecules comprise mouse chromosome 3 and pAA2 DNA sequences flanking the 5' intregation site comprising a nucleic acid junction sequence as set forth in SEQ ID NO: 4 respectively flanking the 3' integration site comprising a nucleic acid junction sequence as set forth in SEQ ID NO: 5.

This invention provides a replicable vector comprising the above nucleic acid. This invention provides a host cell comprising the vector.

This invention provides the use of a nucleic acid of at least 14 nucleotides capable of specifically hybridizing with the nucleic acids of the subject invention.

This invention provides a method of diagnosing inner ear dysfunction in a subject comprising: a) contacting the nucleic acid sample obtained from a subject with the above nucleic acid which binds specifically to the mutated portion of the ysb locus; and d) detecting the labeled nucleic acid, thereby detecting mutant ysb and thereby diagnosing inner ear dysfunction in the subject.

This invention provides a method of diagnosing pigmentation dysfunction in a subject comprising: a) contacting the nucleic acid sample obtained from a subject with the above nucleic acid which binds specifically to the mutated portion of the ysb locus; and b) detecting the labeled nucleic acid, thereby detecting mutant ysb and thereby diagnosing pigmentation dysfunction in the subject.

This invention provides a method of diagnosing cell growth dysfunction, cell proliferation dysfunction, or cell death dysfunction in a subject comprising: a) contacting the nucleic acid sample obtained from a subject with the above nucleic acid which binds specifically to the mutated portion of the ysb locus; and b) detecting the labeled nucleic acid, thereby detecting mutant ysb and thereby diagnosing cell growth dysfunction or proliferation dysfunction in the subject.

The invention provides a method determining vestibular dysfunction in a subject comprising: a) contacting the nucleic acid sample obtained from a subject with the above nucleic acid which binds specifically to a mutated portion of the ysb locus; b) detecting the labeled nucleic acid, thereby detecting gene responsible for yellow coat color, thereby indicating the presence of vestibular dysfunction in the subject.

### Brief Description of the Figures

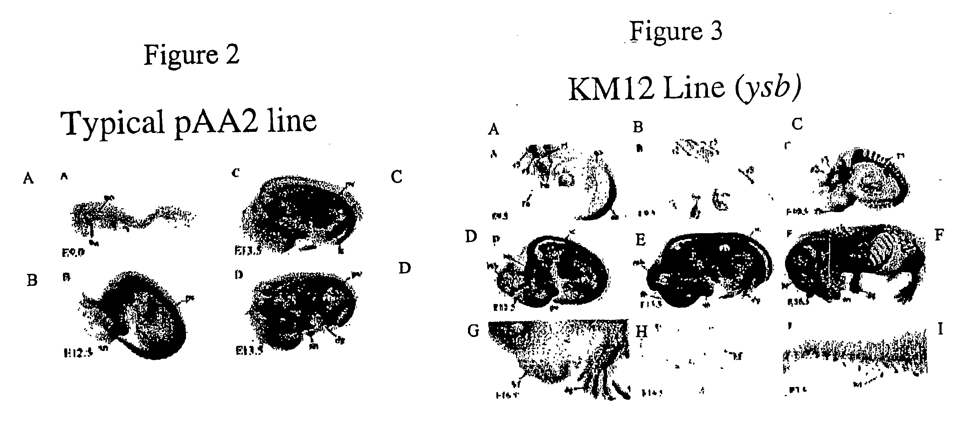

Figure 1
   (A) A homozygous *ysb* mouse showing yellow coat colour compared with (B) a heterozygous littermate, agouti and (C) a black C57BL.
Figure 2
   Whole-mount X-gal staining (blue) of different pAA2 mouse embryos showing the typical LacZ expression pattern at different embryonic stages.
   (A) 9.0 dpc embryo; (B) 12.5 dpc embryo; (C)13.5 dpc, (D), 13.5 dpc embryo. Expression sites include branchial arch (ba), notochord (no), prevertebrae (pv), snout (sn) and digits (dg).
Figure 3
   *LacZ* expression as seen by X-gal staining (blue) of KM12 transgenic embryos at different embryonic stages. *LacZ* is expressed in sites typical for pAA2 (Fig 2) and in extra sites including rhombomeres (r) 2,3 & 5 in hindbrain (hb), neural tube (nt), spinal cord (sc) and hair follicles (hf) which are not the normal expression sites of the Col2a1 transgene. (A), whole-mount X-gal stained 9.5 dpc KM12 embryo shows *LacZ* expression in branchial arch (ba); heart (he); notochord (no); rhombomeres (r) 2.3 and 5; otic vesicle (ov); neural tube (nt). (B) shows sagittal section of 10.5 dpc KM12 transgenic embryo. (C), At 10.5 dpc., KM12 transgenic embryo has additional expression sites at prevertebrae (pv), forebrain (fb) and dorsal root ganglia (drg) (not shown in this view) but with the disappearance of r5 expression. (D) expression pattern of 12.5 dpc fetus, at this stage staining is also in midbrain (mb), hindbrain (hb), spinal cord (sc). In (E), *LacZ* expression in 13.5 dpc embryos is also seen in snout (sn), digits (dg) and ribs (rb). (F) At 16.5 dpc, expression in the brain and vertebrate continue to be seen in sagittally-halved fetus. Expression is seen in hair follicles in whole-mount (G), and sectioned (H) 16.5 embryos, this continues to be present in 3.5 days postnatal skin which is whole-mount x-gal stained and cleared (I).
Figure 4
   Swimming test: A), a wild-type mouse - floats and swims in water; B),C),D), and E) a homozygous yellow submarine (ysb) mouse - circles and submerges in water
Figure 5
   Reaching response test. Mouse on left, a ysb homozygote with vestibular dyfunction, showing a tendency to curl up towards the belly when held by tail. Mouse on right, a normal reaching response, with outstretched body.
Figure 6
   Southern blot hybridization of genomic DNA from mice heterozygous (he) and homozygous (ho) for the pAA2 transgene in the KM12 line and non-transgenic (nt) control littermates using (A) kreisler, (B) agouti, and (C) α-MSHR cDNA probes. The structural genes of kreisler, agouti and α-MSHR have not been disrupted by COL2A1-lacZ transgene in *ysb* mice.
Figure 7
   The ysb locus and chromosomal localisation of the pAA2 transgene. (A) Localisation of pAA2 (cheah et al, 1995) in KM12/ysb transgenic mice to chromosome 3 by fluorescence in situ hybridization (FISH) using pAA2 as probe. The transgene maps to A2 and B-C region on metaphase ysb chromosomes.(B) Chromosomal map of transgenic integration.(C) Genomic clones isolated by PCR probes for integration sites 1 and 2. (D) Chromosomal localisation of genomic clones for integration sites 1 and 2 by FISH to wild-type metaphase chromosomes. Both map to A3 on chromosomes 3. Reference: K.S.E. Cheah, A Levy, P.A. Trainor, A.W.K. Wai, T. Kuffner, C.L. So, K.K.H. Leung, R.H. Lovell-Badge and P.P.L. Tam. (1995) Human COL2A1-directed SV40 T-antigen expression in transgenic and chimeric mice results in abnormal skeletal development. J.Cell Biol. 128 223-237.
Figure 8
   Fluorescence *in situ* hybridisation (FISH) of *ysb* chromosomes using transgene (pAA2) as probe. (A) The transgene has integrated into two insertion sites in the chromosome. Arrow shows position of fluorescence signals.(B) DAPI banding pattern shows that pAA2 insertion sites correspond to chromosome 3. (C) Corresponding bands that show fluorescence signal is shown by dots.
Figure 9
   Whole-mount in situ hybridization of 9.5 dpc embryos using Krox-20 riboprobe, double-stained for β-galactosidase activity (reddish). Homozygous ysb embryos (E,F) and heterozygous ysb embryos (C,D) have normal Krox-20 expression pattern in rhombomeres (r) 3 and 5 at 9.5 dpc. Sagittal view (A), and dorsal view (B) of non-transgenic littermate showing normal pattern.
Figure 10
   Whole-mount neurofilament immunostaining of 10.5 dpc non-transgenic control (A,B), heterozygous *ysb* (C,D), and homozygous ysb (E,F) embryos, using monoclonal antibody (2h3), double-stained for β-galactosidase activing. A reduction of the eighth nerve (VIIIn) is observed in homozygous ysb at 10.5 dpc.
Figure 11
   3-D reconstructed images of inner ears of a)non-transgenic, b) heterozygous *ysb,* and c) homozygous *ysb* 16.5 dpc fetuses showing structural malformations in developing homozygous ysb inner ears. Note superior semicircular canal is obliterated (*) in homozygous *ysb.*
Figure 12
   3-D reconstructed images of inner ears of 16.5 dpc. fetuses a) wild-type non-transgenic (+/+), b)heterozygous ysb (+/-) and c) homozygous ysb (-/-). Note that neuroepithelial structures are missing in developing inner ear of homozygous ysb fetuses. In 16.5 dpc homozygous ysb (-/-), superior semicircular canal is partially obliterated* and ends in a blind sac (c); the utricular macula, superior and lateral cristae are missing.
Figure 13
   3-D reconstructed images of inner ears at 13.75 dpc showing structural malformations in developing homozygous ysb inner ears. Superior semicircular canal is obliterated* in homozygous *ysb.*
Figure 14
   A) Sequence of PCR product at integration site 1
   B) Sequence of PCR product at integration site 2
Figure 15
   Southern analyses of *ysb* genomic DNA using flanking probes at integration sites 1 and 2. Different size bands are observed in the mutant alleles when probed with flanking probes from both integration sites (shown by corresponding arrows). No segregation of the two integration sites are observed in the *ysb* mice studied (6^{th} generation). Wt, wild-type; he, heteozygous *ysb*; ho, homozygous *ysb*
Figure 16
   Deletion in ysb at integration site 2.
   (A) 20 kb deletion in *ysb.* B-E; Autoradiograms of Southern blots of genomic DNA digested with EcoRI (B,D), SacI (C) and Xbal (E) from *ysb* heteozygote, homozygote and non-transgenic mice. Probes were: B), pSD14 (1.4kb EcoRI); C), pSD11 (1.9kb SacI); D), pSD9 (3.2kb BamHI) ; E) pSD8 (2.0kb
      Sac I)
   (B) Note rearrangement band is observed when pSD14 (1.4kb EcoRI) is used to probe homozygous *ysb* genomic DNA.
   *signifies homozygous ysb
   Note deleted sequences revealed by probes pSD11(C); pSD9 (D); and pSD8 (E)
Figure 17
   Sequences information for the 8.1 kb within *ysb* locus deletion.
   Locus: chromosome 3
   Definition: pPL5
   Keywords: genomic DNA
   Source: house mouse
   Organism: Mus Musculus
   Vector: pBluescript
   Inset: 8114 bp
   Insertion sites: Hind III
   Information:
   A. Sequence of pPL5 containing 8114 bp DNA fragment (Hind III cut) of 20 kb deleted sequences in integration site 2
   B. Sequence alignment showing pPL5 (1743 bp-1906 bp) contains sequences 90% identity to IMAGE 1196866
   C. Sequence alignment showing pPL5 (106-395 bp) contains sequences with 100% identity to IMAGE 636095
Figure 18
   Summary map of pPL5 showing location of EST-IMAGE sequences

### Detailed Description of the Invention

This invention provides an isolated nucleic acid which defines a mutant yellow submarine locus, wherein the mutant yellow submarine locus is identical to a wildtype yellow submarine locus except for an integration of a pAA2 transgene into at least one region on a chromosome.

These isolated nucleic acid molecules comprise mouse chromosome 3 and pAA2 DNA sequences flanking the 5' intregation site comprising a nucleic acid junction sequence as set forth in SEQ ID NO: 4 respectively flanking the 3' integration site comprising a nucleic acid junction sequence as set forth in SEQ ID NO: 5.

The isolated nucleic acid of the subject invention includes genomic DNA, RNA, cDNA. The nucleic acid may be labeled with a detectable marker. The detectable marker includes but is not limited to a radioactive, a colorimetric, a luminescent, or a fluorescent label.

This invention provides a replicable vector comprising the above nucleic acid. This invention provides a host cell comprising the vector. In one embodiment, the cell is a eukaryotic cell. In one embodiment, the cell is a bacterial cell. The vectors of the subject invention include but are not limited to a plasmid, cosmid, λ phage, YAC, BAC, or PAC.

This invention provides a method of diagnosing inner ear dysfunction in a subject comprising: a) contacting the nucleic acid sample obtained from a subject with the above nucleic acid which binds specifically to the mutated portion of the ysb locus; and b) detecting the labeled nucleic acid, thereby detecting mutant ysb and thereby diagnosing inner ear dysfunction in the subject.

This invention provides a method of diagnosing pigmentation dysfunction in a subject comprising: a) contacting the nucleic acid sample obtained from a subject with the above nucleic acid which binds specifically to the mutated portion of the ysb locus; and b) detecting the labeled nucleic acid, thereby detecting mutant ysb and thereby diagnosing pigmentation dysfunction in the subject.

This invention provides a method of diagnosing cell growth dysfunction, cell proliferation dysfunction, or cell death dysfunction in a subject comprising: a) contacting the nucleic acid sample obtained from a subject with the above nucleic acid which binds specifically to the mutated portion of the ysb locus; and b) detecting the labeled nucleic acid, thereby detecting mutant ysb and thereby diagnosing cell growth dysfunction or proliferation dysfunction in the subject.

In one embodiment of the above method, the subject is mammal. In another embodiment, the subject is a non-mammal. The subject mat be a human, a primate, an equine, an opine, an avian, a bovine, a porcine, a canine, a feline or a murine. The subject may be a vertebrate.

The invention provides a method determining vestibular dysfunction in a subject comprising: a) contacting the nucleic acid sample obtained from a subject with the above nucleic acid which binds specifically to a mutated portion of the ysb locus; b) detecting the labeled nucleic acid, thereby detecting gene responsible for yellow coat color, thereby indicating the presence of vestibular dysfunction in the subject.

### Experimental Details

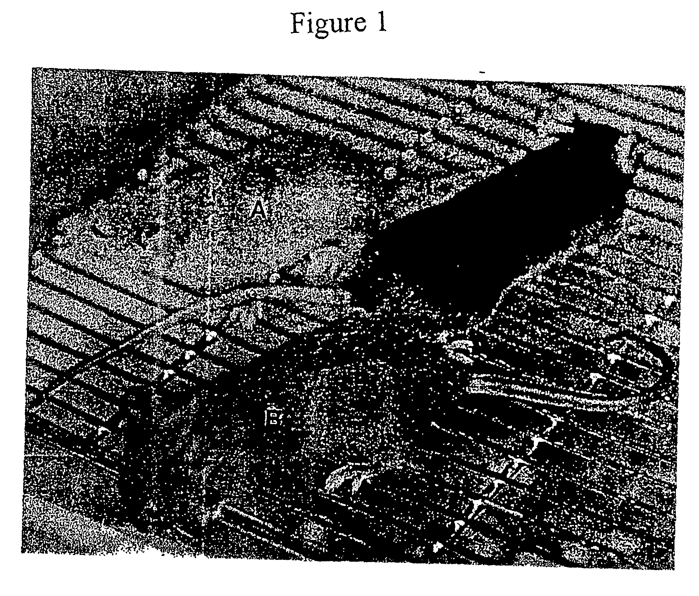

A mouse line (KM12) bearing a recessive mutation, caused by insertion of a transgene was created. These mice are characterized by a change in coat colour to yellow and circular behaviour. (Figure 1)

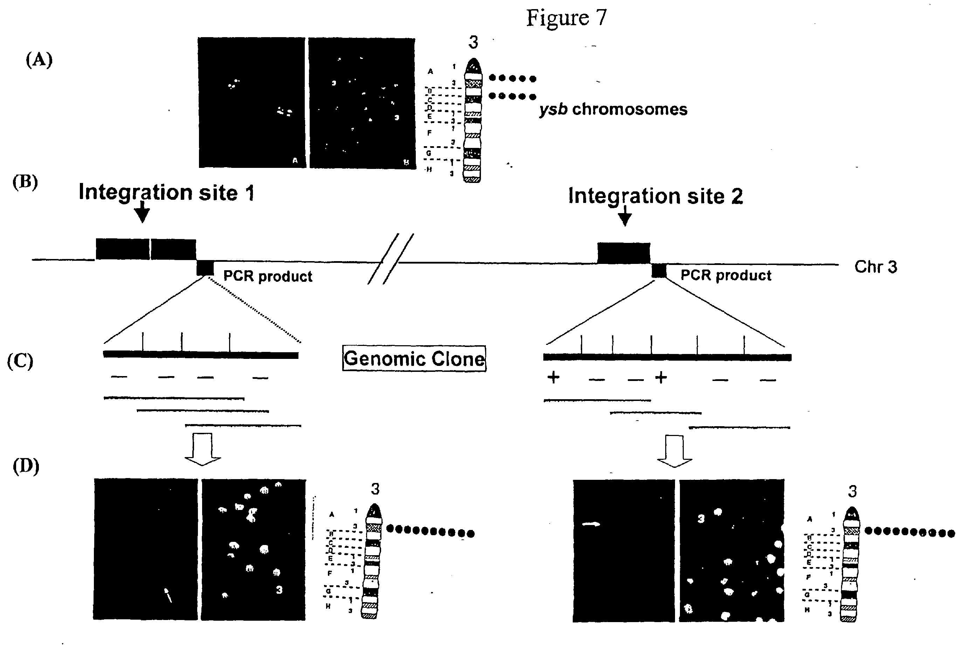

The yellow coat of KM12 mice suggests abnormal regulation of pigment synthesis. Hair pigmentation in mice is mediated by many developmental and signalling processes involving enzymes, transcription factors, a growth factor and its receptor, a membrane transport protein, a hormone receptor and an antagonist of hormone binding. In mice, wild-type colour hair is agouti and contains two pigments in which the bases and tips contain the black pigment eumelanin and an intermediate band containing phaeomelanin. Two genes in mice, *agouti* (*A*) on chromosome 2 and extension (*E*) on chromosome 8 are involved in the regulation of the relative amounts of eumelanin and phaeomelanin in the mouse. Mutations in these two loci result in a yellow coat colour in mice. Dominant mutations in the *A* gene, such as lethal yellow and viable yellow, result in an obese mouse which is completely or almost all yellow because only phaeomelanin is made. In contrast mutations in *E* which result in yellow coat colour (*e*) are recessive. Both the *agouti* and *extension* loci have been cloned. The *A* gene encodes a 131 amino acid polypeptide with a structure consistent with its proposed paracrine function. *E* encodes the (-MSH receptor (MSH-R). It has been established that neither *A* nor *E* have been mutated in KM12 mice. In addition, the transgene has been mapped to two integration sites on mouse chromosome 3 (Figure 7), consistent with a mutation outside the *A* and *E* loci.

The circular behaviour of KM12 mice suggests improper inner ear function which affects balance. The mature inner ear consists of the auditory apparatus, which is responsible for the perception of sound (organ of Corti) and the vestibular apparatus which is responsible for the sense of balance. Many genes have been found to regulate the development and functions of the inner ear and mutations which cause hearing and balance defects have been found in both human and mouse. These genes encode diverse classes of proteins such as transcription factors, secreted growth factors, signalling molecules, receptors, cytoskeletal components, intracellular transporters, and ion channel proteins. Although pigmentation defects associated with deafness have been found (such as *micropthalmia, dilute*) to date no mutations causing both yellow coat and inner ear defects have been identified in mice.

The databases for candidate genes and known mutant loci on chromosome 3 which may account for the yellow coat and vestibular dysfunction were searched and none were found none. The mutation is therefore a novel one. Based on the phenotypic features of KM12 homozygous mice, the mutant locus has been named *yellow submarine (ysb).*

To define the molecular defect(s) in *ysb* mice, a necessary prerequisite is to identify the mutated gene(s) and characterise the phenotypic abnormalities. In the current RGC project Analysis of the vestibular abnormalities in *ysb* mice has been started. Abnormalities in the acoustic nerve and inner ear have been found in *ysb* embryos. Inverse PCR was used to isolate flanking DNA from both the transgene insertion sites. These flanking DNA have been used to isolate genomic clones spanning a total of 49kb DNA. Southern analyses using these clones show that approximately 20kb DNA has been deleted at integration site 2. Clones for both integration sites have been mapped to the same site on chromosome 3. The data suggest that the transgene integration has caused a deletion and chromosomal inversion. It has also been determined that a chromosome 3 recessive mutant (*named lcc: light coat and circling*) with features similar to *ysb,* which arose as a result of X-ray irradiation and which may be allelic to *ysb* (Dr. C. Tease (MRC Mammalian Genetics Unit, Harwell) and a comparison of the two mutants is being performed. Intercrosses between *ysb* and *lcc* mice show that the *ysb* mutants cannot complement the *lcc* mutation strongly suggesting the mutations are allelic.

Molecular genetics, bioinformatics, developmental biology, transgenic and physiological approaches are used to identify the gene(s) at the +^{*ysb*} locus and study the molecular and developmental bases underlying the defect(s) in *ysb* mice. The following methods are used : 1) isolation and characterization of the +^{ysb} gene(s); 2) determination of the nature of the *ysb* mutation; 3) performing genetic complementation tests between *ysb* and *lcc* mice, 4) performing transgenic rescue experiments, 5) characterization of the developmental defects in the inner ears of *ysb* mice using 3-D reconstruction analyses, molecular markers and chimera studies; 6) characterization of the neurophysiological changes in balance and hearing in *ysb* mice. Although some emphasis was placed on the inner ear defects of *ysb,* by cloning and characterizing the *ysb* gene(s) insight is gained into the biochemical and possible intracellular signal transduction defect(s) underlying the coat colour change. Approximately 1/1000 infants are affected by hearing defects at birth, two thirds of which have a genetic basis. About half of children with hearing impairment also have vestibular dysfunction. Mice are good models for studying auditory defects because of the similarity in structure and development between mouse and human inner ears. These studies provide fundamental information on the mechanisms of inner ear development and the molecular basis by which inner ear defects may arise in balance disorders of mice and human.

### The KM12 mouse line

In making transgenic mice sometimes integration of the exogenous DNA disrupts the function of one or more genes. While studying the regulation of the *COL2A1* gene, using a recombinant plasmid (pAA2) which contained regulatory DNA sequences from *COL2A1* linked to the *lacZ* reporter gene (Cheah et al. 1995), a recessive mutation, caused by insertion of the transgene, was created. These mice are characterized by a change in coat colour to yellow and circular behaviour, only seen in offspring homozygous for the transgene (Fig. 1, Appendix). In developing KM12 embryos the transgene is expressed not only in the sites expected for *COL2A1* but also in additional expression domains such as specific rhombomeres of the hindbrain (r2,3,5), the spinal cord, dorsal root ganglia, and in the hair follicles of the skin. These neural and skin sites of expression are not typical of the endogenous *Col2a-1* gene but are consistent with the coat colour and behavioural phenotype of KM12 mice (Figures 2,3).

### Agouti and yellow coat colour

The yellow coat of KM12 mice suggests abnormal regulation of pigment synthesis. In mice, wild-type color hair is agouti and contains two pigments in which the bases and tips contain the black pigment eumelanin and an intermediate band containing phaeomelanin. The regulation of hair pigmentation in mice is mediated by many developmental and signalling processes involving several enzymes, transcription factors, a growth factor and its receptor, a membrane transport protein, a G protein-coupled hormone receptor and an antagonist of hormone binding. These pigments are synthesized in melanocytes by tyrosinase (reviewed in) . Two genes in mice, *agouti* (*A*) on chromosome 2 and extension (*E*) on chromosome 8 are involved in the regulation of the relative amounts of eumelanin and phaeomelanin in the mouse. Mutations in these two loci result in a yellow coat colour in mice. Dominant mutations in the *A* gene, such as lethal yellow and viable yellow, result in an obese mouse which is completely or almost all yellow because only phaeomelanin is made. In contrast mutations in *E* which result in yellow coat colour (*e*) are recessive. Both the *A* and *E* loci have been cloned. The *A* gene encodes a 131 amino acid polypeptide with a structure consistent with its proposed paracrine function. *E* encodes the (-MSH receptor (MSH-R), a 35kD polypeptide with seven transmembrane domains and is expressed in melanocytes. Activation of MSH-R promotes eumelanin synthesis while agouti protein enhances phaeomelanin synthesis.

### Genes and inner ear defects

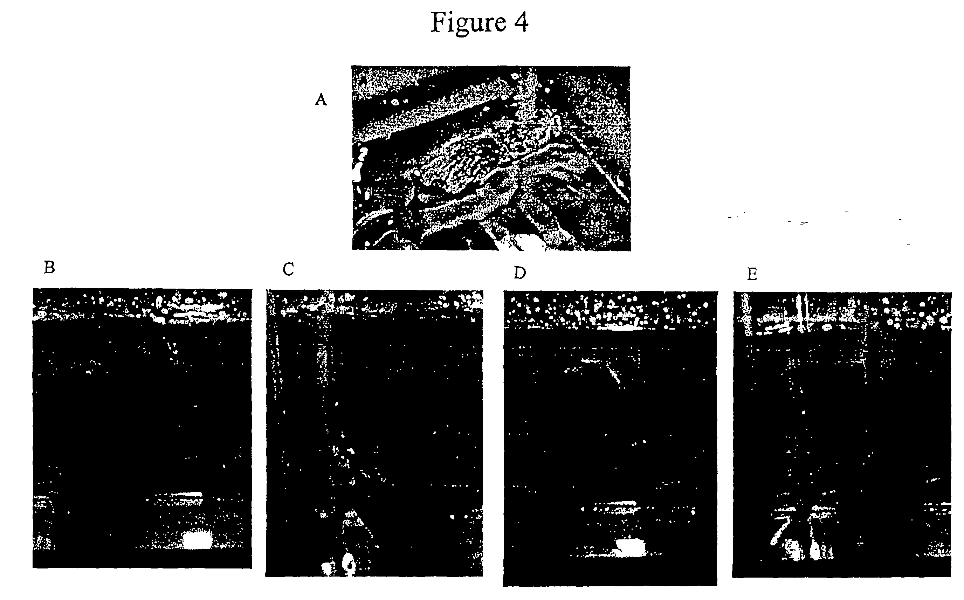

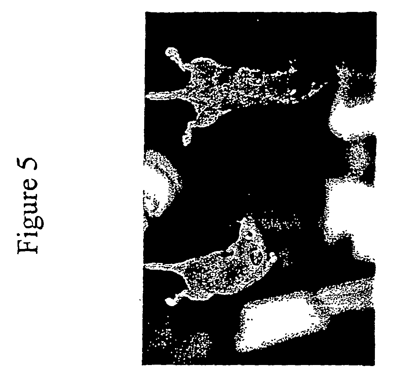

Many deaf mouse mutants are characterized by the classic shaker/waltzer behaviour of circling, head tossing and hyperactivity. The circular behavior of KM12 mice suggests improper inner ear function which affects balance and hearing. KM12 mice also show other characteristics of inner ear defects such as head-tossing, and an inability to swim (Figure 4). In addition KM12 mice show an abnormal reaching response. When picked up by the tail, KM12 mice do not stretch out their limbs as normal mice do, but rather curl up towards their bellies (Fig.5, Appendix). Although KM12 mice show some response to sharp sounds (Preyer reflex), this does not appear as strong as for wild-type mice. Therefore it is possible that KM12 mice are partially hearing impaired. Physiological tests which measure endocochlear potential (EP) and compound action potential (CAP) show that *ysb* mice are deaf and that *ysb*/*lcc* compound heterozygotes are profoundly deaf. These results suggest that the stria vascularis is not functioning properly and cochlear nerve activity in response to a sound is also abnormal.

The inner ear is a complex sensory organ which develops from a single-cell-layered epithelium (otic placode) which invaginates by a series of cell and tissue movements and closes to form the otic vesicle. Further morphogenetic movements and multi-step inductive events, which regulate differentiation and proliferation, lead to the formation of the organ for hearing and balance. The mature inner ear consists of the auditory apparatus, which is responsible for the perception of sound (organ of Corti) and the vestibular apparatus which is responsible for the sense of balance. Many genes have been shown to be expressed in the developing inner ear. These include genes encoding transcription factors (e.g. *Nkx5.1*/ *hmx3, Nkx5.2*/*hmx2, otx-1, msx1, pax2, kreisler,* etc); secreted factors (e.g. *Bmp4, fgf3, fgf2, bdnf*), receptors (e.g. *EphA4, trkA,* trkB, *trkC, Ednrb, PTHrpR*), cytoskeletal proteins such as unconventional myosins (e.g. *Myo7a, myo15*).

Inner ear defects have been traced to one or more of three types of abnormalities: morphogenetic, cochleo-saccular, and neuroepithelial. Morphogenetic defects are caused by developmental abnormalities in structure of the inner ear (labyrinth). Cochleo-saccular abnormalities result from defects in the secretory epithelium of the cochlear duct. Neuroepithelial defects arise from failure of the sensory epithelia to complete normal maturation. In addition abnormal hindbrain development may result in vestibular dysfunction. For example, *kreisler* mice are characterized by deafness and circular behaviour in adults, and in the embryo, by abnormalities in the positioning of the otic vesicles and segmentation of the hindbrain.

Mice are good models for the studying auditory defects because of the similarity in structure and development between mouse and human inner ears. Mutations in several genes expressed in the inner ear have been shown to cause hearing and/or balance disorders in human and mouse. These include *Myo7a, Myo15, kreisler, hmx3 (nkx5.1), fgfr3* and others.

The following methods are performed: a) characterize abnormalities in hindbrain and ear development in KM12 mice and b) isolate the DNA sequences at the site(s) of integration of the transgene in KM12 mice.

### Yellow submarine: a novel mutant locus

The recessive nature of the mutant phenotype suggests that the transgene has not integrated into the *A* gene.

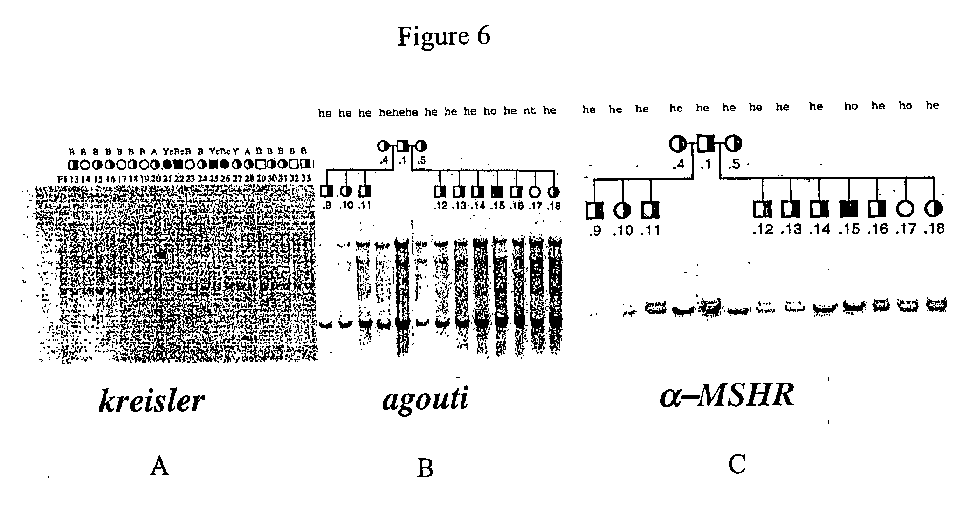

Insertion of the transgene into the coding sequences of MSH-R, ACTH-R, agouti and *Kreisler* genes has been tested because they have been shown to be important for agouti coat colour or inner ear development. Southern blot analyses of DNA of KM12 mice, using probes for the *Kreisler, agouti, E* and ACTHR (*Mc2r*) genes, [gifts of Dr. G. Barsh (UCSF), Dr. R. Woychik (Oak Ridge National Laboratory, Oak Ridge) and Dr. R. Cone (Oregon Health Sciences University, Portland)], show that the coding sequences of these genes have not been interrupted by the transgene (Figure 6). These results therefore exclude chromosomes 2 (*agouti*, *kreisler*)*,* 8 (*E*), and 18 (*Mc2r*).

Using the whole transgene and its end fragments as probes in Southern blot analyses, we have determined that 3 copies of the transgene have integrated into 2 sites in the genome. There are two copies of the transgene, head to tail, at one integration site (Int.1) and one copy at the other (Int2) (Summarized in Fig 7, Appendix). However, since the coat colour and behavioral phenotypes have not segregated over approximately 176 meioses (6 generations), the transgene may have caused a rearrangement and/or deletion of part of the chromosome. Therefore the coat coloor and behavioral phenotypes of KM12 mice could be caused by mutation of one or more genes.

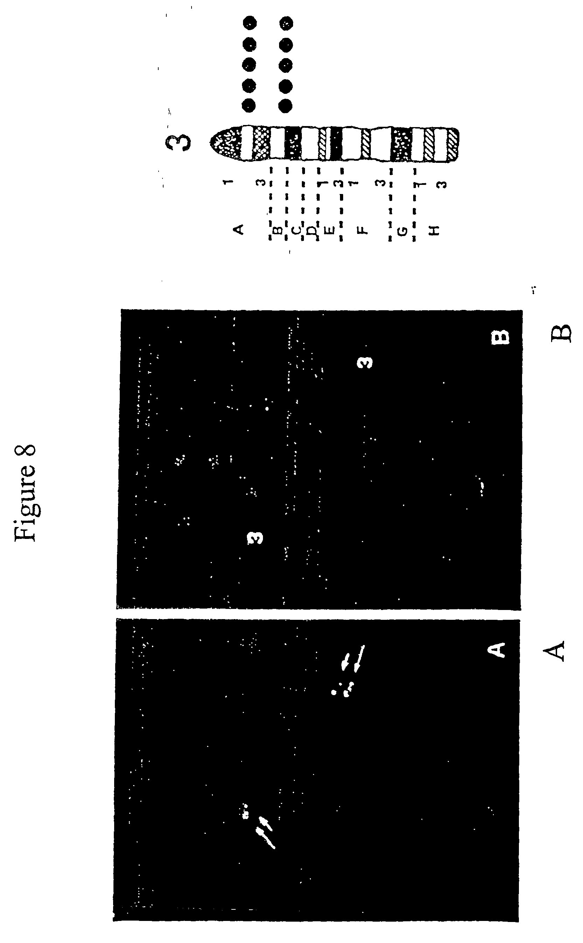

The chromosomal assignment of the KM12 transgene is an essential and informative first step in defining the nature of the mutation. The transgene has been mapped by FISH to two integration sites on mouse chromosome 3, consistent with the Southern analyses (Figure 7,8). The data are also consistent with the results excluding the *A, E, Kreisler* loci.

To date no mutations causing both yellow coat and inner ear defects have been identified in mice. The databases have been searched for candidate genes and known mutant loci on chromosome 3 which may account for the yellow coat and vestibular dysfunction and found none. The mutation is therefore a novel one. Based on the phenotypic features of KM12 homozygous mice, the mutant locus has been named *yellow submarine (ysb)* (and homozygous mutants hereafter referred to as *ysb,* wild-type as +^{ysb} ; Leung, K.K, S. Dong, A. Tang, H. Heng, L.C. Tsui, P.P.L. Tam & K.S.E. Cheah *Yellow submarine (ysb)* a newly discovered locus regulating hair colour and inner ear function. Manuscript in preparation)

### Characterisation of ysb phenotype

*Ysb* mice are a valuable model to identify molecules involved in controlling pigmentation and balance. The structural abnormalities in *ysb* mice have been analyzed. The phenotype and pattern of expression of the transgene in *ysb* mice indicate that integration of the transgene has caused a recessive mutation which affects the regulation of pigmentation and also causes abnormal inner ear development. The initial studies have focused on the inner ear abnomalities in *ysb* mice.

### Hindbrain and cranial nerve structure

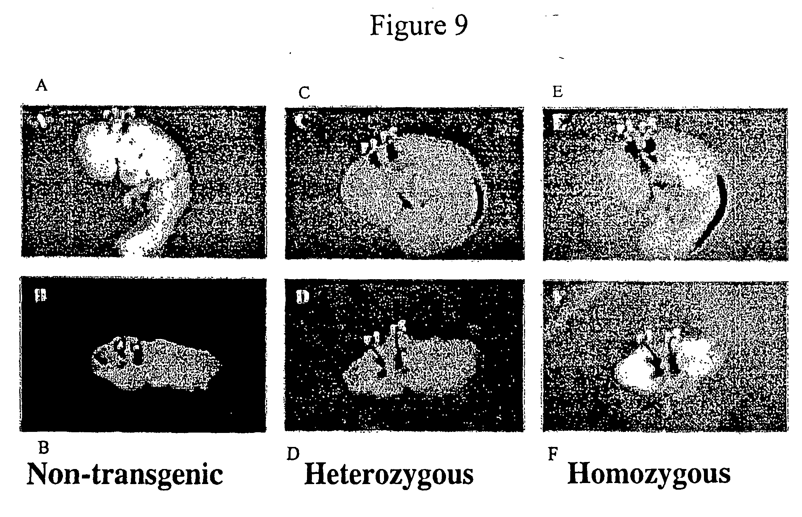

In vertebrate embryogenesis, the process of segmentation in which reiterating blocks of tissue form along the anterior-posterior body axis, are fundamental to pattern formation and differentiation. In the development of the hindbrain, segmentation occurs with rhombomere formation in the neural epithelium. A detailed study of *kreisler* embryos using markers for hindbrain segementation such as *Krox-20,* and *Hox* genes have shown that the *kreisler* mutation is probably due to the consequence of abnormal segmentation of the hindbrain resulting in the loss of rhombomeres (r) 5 and 6. *Krox-20* is a useful marker for hindbrain segmentation being normally expressed in r3 and 5 at 9.0-9.5 days, and *lacZ* expression was found in r3 of *ysb* embryos. In order to assess if the phenotype of *ysb* mice is associated with a problem in hindbrain development, initially the pattern of expression of *Krox-20* was studied in heterozygote and homozygote 9.0-9.5 day embryos by *in situ* hybrization in whole mount and on sections and compared to that for wild-type. Whole-mount in situ hybridization showed no alterations in the rhombomere expression of *Krox-20* mRNA in homozygotes and heterozygotes at 9.5 dpc (Figure 9). This is unlike the *Kreisler* mutant suggesting that the mutation has not caused gross alterations in rhomomeres 3 and 5.

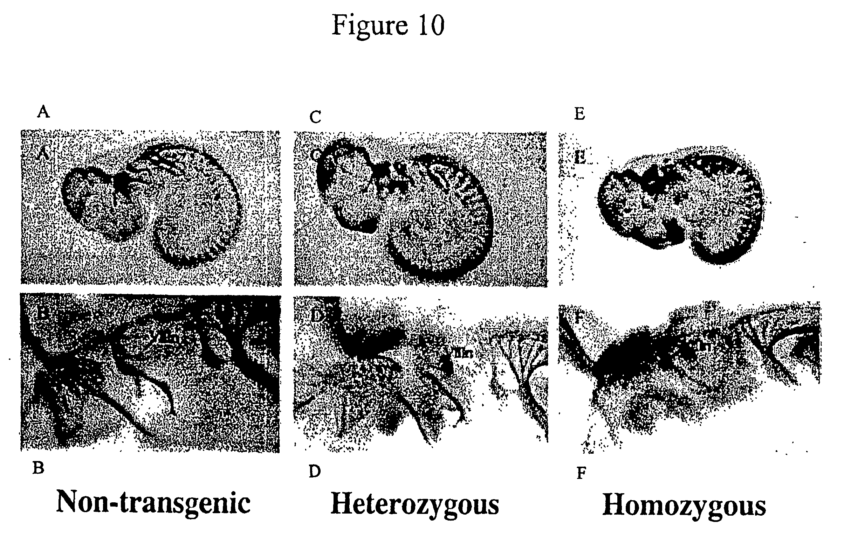

Whole-mount immunostaining studies on 10.5 day *ysb* embryos using an antibody (2H3) against neurofilament, showed a reduction of the VIIIth nerve (which gives rise to the vestibular nerve) in homozygote mutants, while the other cranial nerves appeared normal (Fig.10, Appendix) . Heterozygotes appeared normal, although the VIIIth nerve seemed to be thinner compared to wild-type. This result is consistent with the vestibular dysfunction in the *ysb* mice. The mutation could therefore have a) affected the ability of the VIIIth nerve to grow towards the otic capsule; b) caused a failure of the full number of progenitors to migrate from the otocyst initially; or c) caused abnormal cell death (apoptosis).

### Inner ear morphology

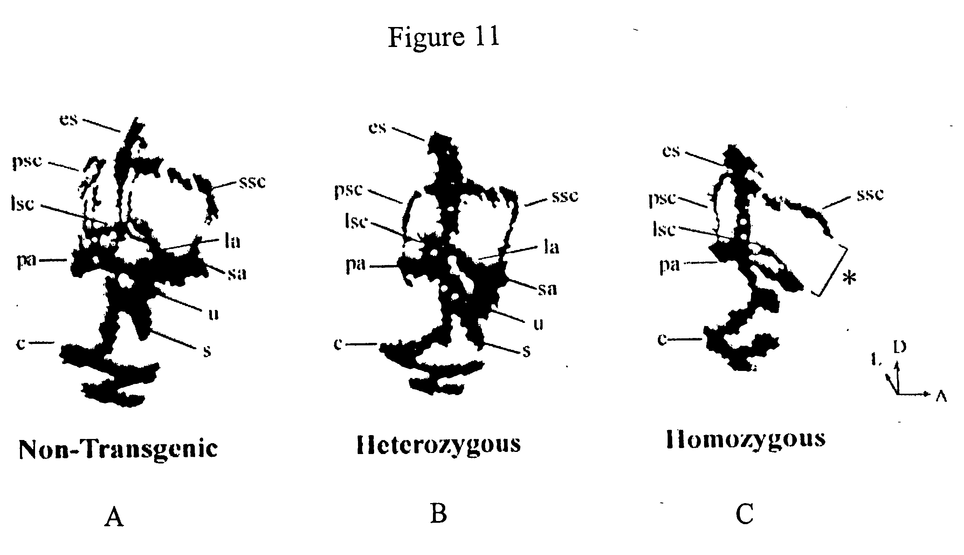

To determine whether there are any inner ear malformations in homozygous ysb mice, 3-D reconstructions of the inner ear of the mice were generated using histological serial sections. 3D reconstructions of images of sections of the ears of wild-type, heterozygous and homozygous ysb fetuses at 16.5 dpc (days post coitum) were carried out. This was followed by painting in the lumen to give an impression of the whole structure of the labyrinth. The data show superior and lateral canal and ampulla defects in two ysb fetuses, with one more severely affected than the other (Appendix, Fig.11).

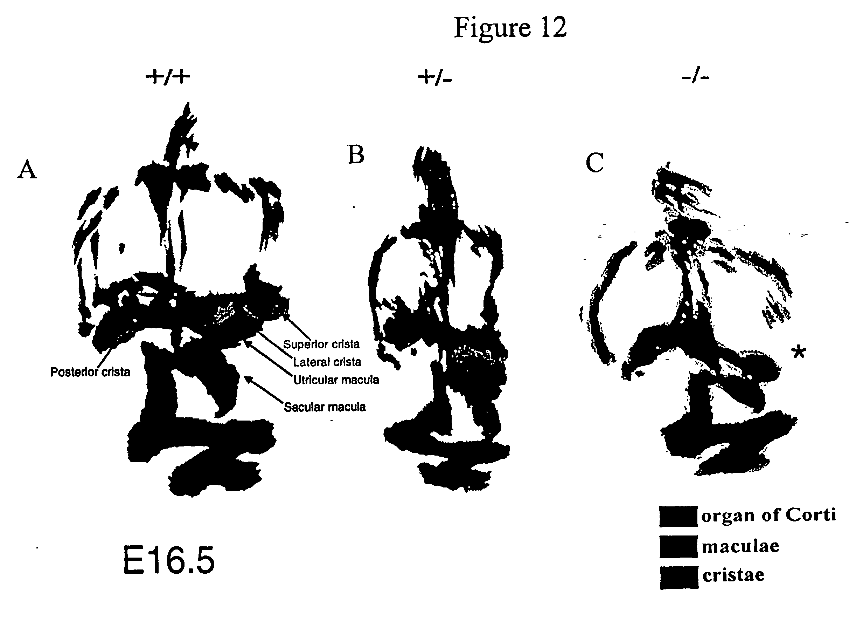

The reconstructions of the lumen in two 16.5 dpc homozygote *ysb* fetuses show the superior and lateral canal were truncated and their ampullae were absent. At 16.5dpc the sensory regions are distinguishable from non-sensory regions because the cells have differentiated into a pseudostratified epithelium. This epithelium appears thicker when compared to the surrounding epithelium because of the two or more layers of nuclei. Because this difference could be observed easily with the light microscope, these thickened areas were traced and painted onto the lumen of ears that had previously been reconstructed (Fig.12, Appendix). The different types of sensory areas including the organ of Corti (detector of sound), the maculae (detectors of linear motion), and the cristae (detectors of rotational motion), are represented in different colours to demonstrate more easily which sensory areas were affected.

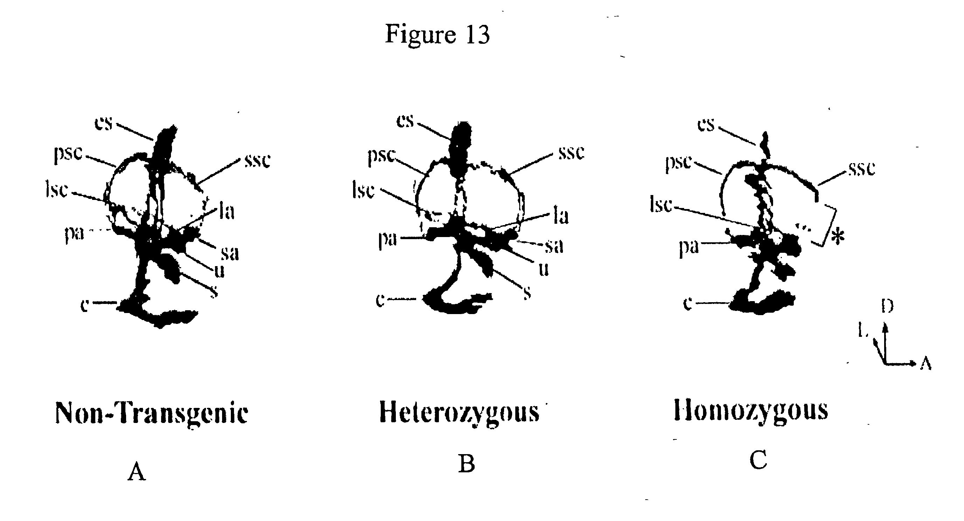

Addition of the sensory information to these reconstructions showed that several different types of sensory areas were abnormal in the homozygote. The superior and lateral cristae which reside in the ampullae were absent (Fig 4, Appendix). This observation is consistent with the previous observation that the ampullae were absent. The detailed analysis of sensory epithelia also showed that there was no ectopic formation of these cristae. Both maculae also appeared to be affected by the mutation: the utricular macula showed almost no thickening in the expected region and while the saccular macula did demonstrate some thickening, it appeared abnormal. The only vestibular patch that appeared normal was the posterior crista. The hearing organ, the organ of Corti, also appeared normal at this time of development. No abnormalities were observed in the heterozygote using this method of analysis, and the wild-type (+/+) littermate was used as a control. Reconstruction of sections from 13.75 dpc fetuses showed a similar defect in the semicircular canals (Figure 13).

### Isolation & characterisation of transgene integration sites

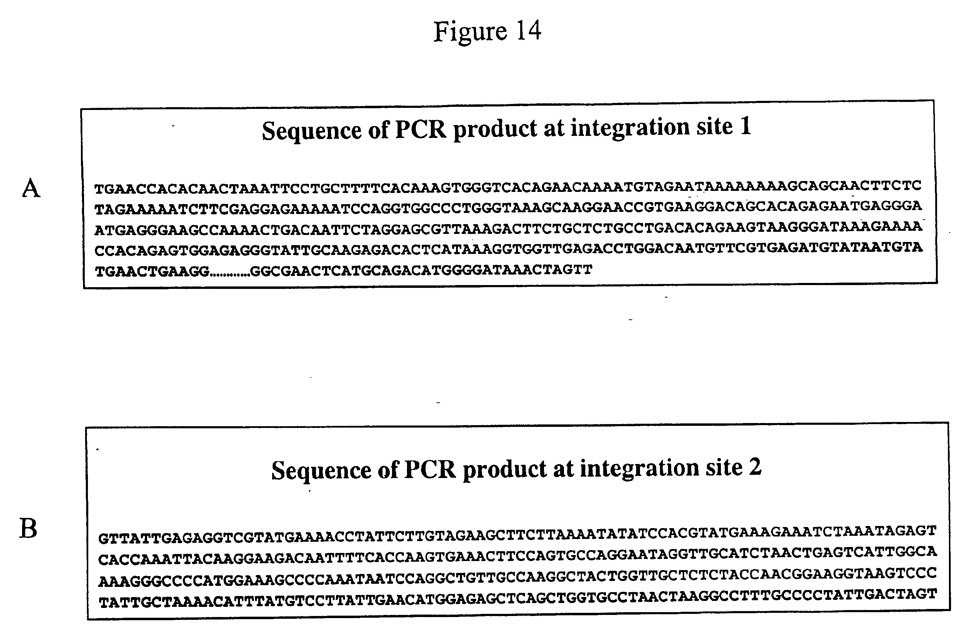

Fig. 7 summarizes the progress in isolating the wild-type locus at the site of transgene integration. By priming within the transgene, inverse-PCR was used to isolate DNA sequences flanking the integration sites (Fig 7). Two PCR products, 350bp (SpeI-1) and 600bp (SpeI-2), were obtained for integration sites 1 and 2 respectively. Sequence analysis (Figure 14) showed the presence of a possible polyA attachment signal in SpeI-1 but a BLAST search of the genomic and EST databases did not reveal significant matches. Longer 5' flanking sequence have been obtained for integration site 1 (1.8kb) and 3' flanking sequence for site 2 (500bp).

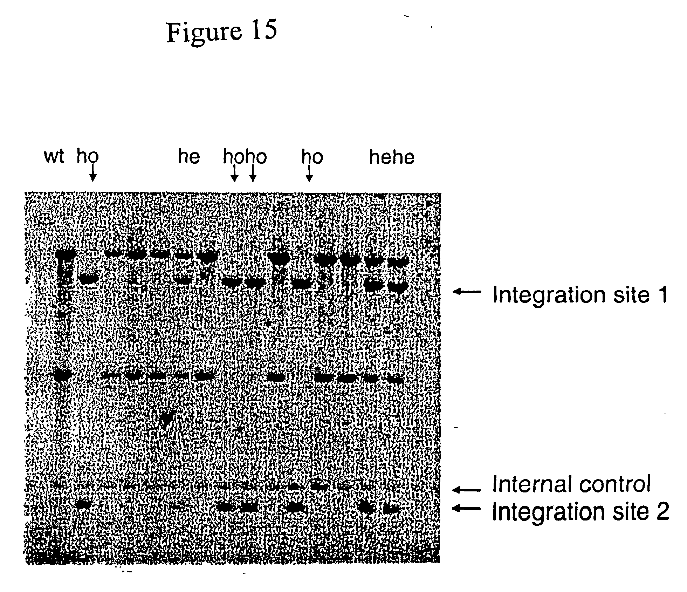

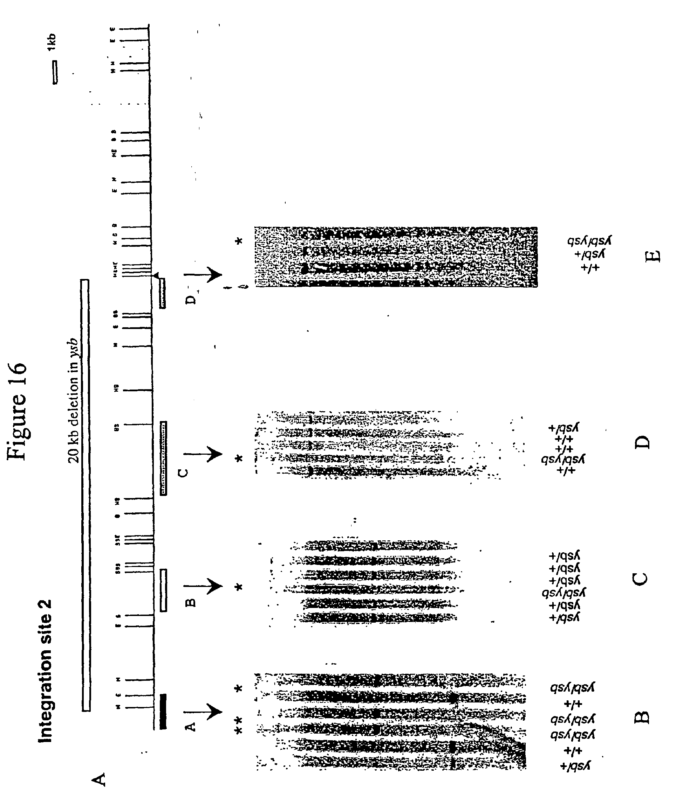

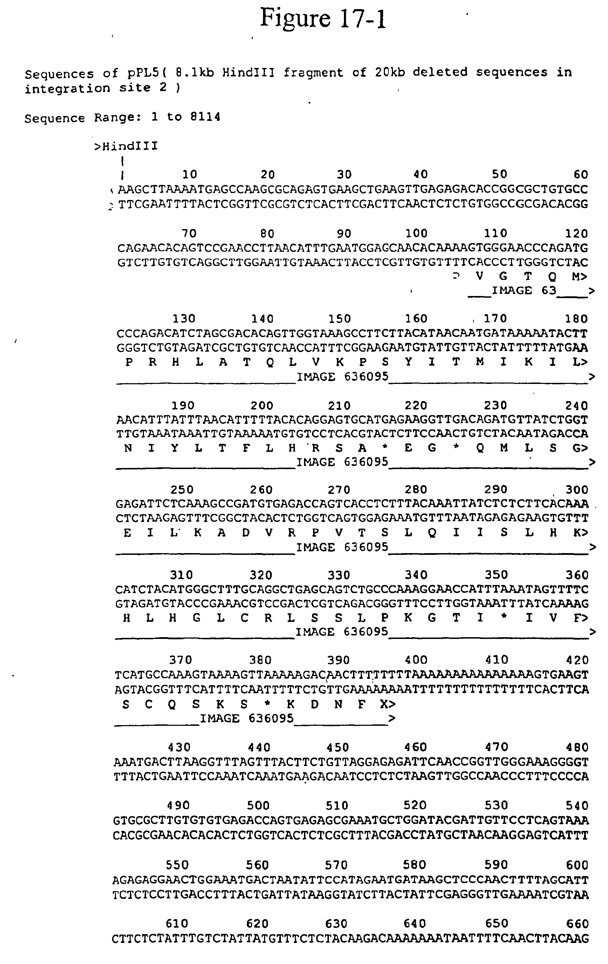

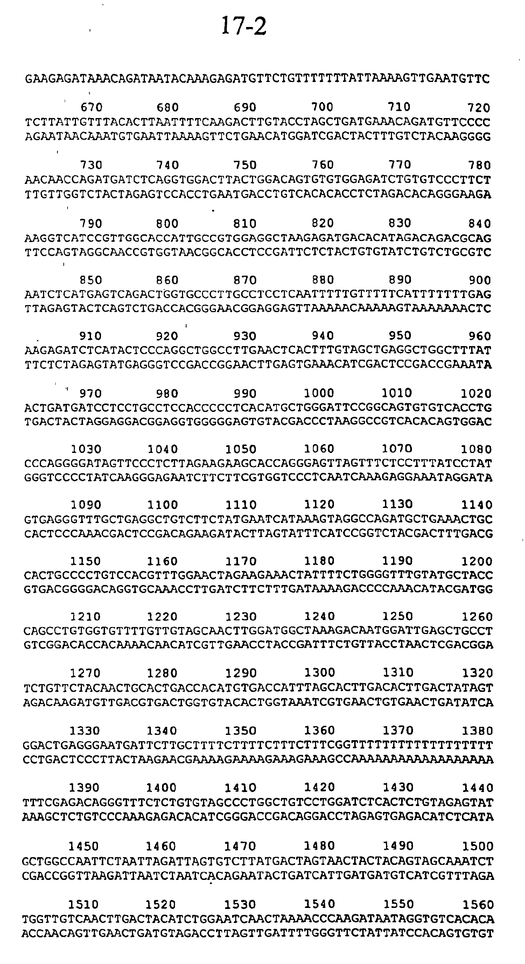

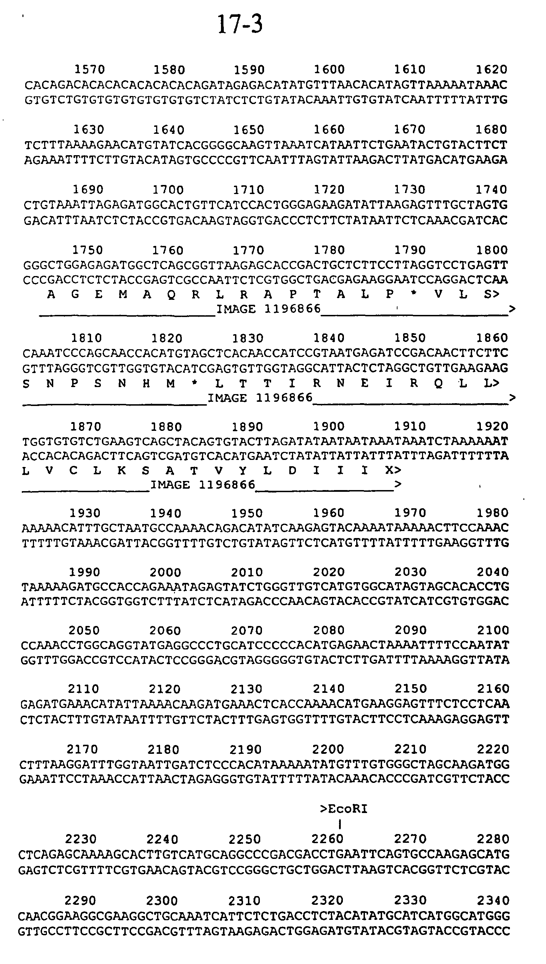

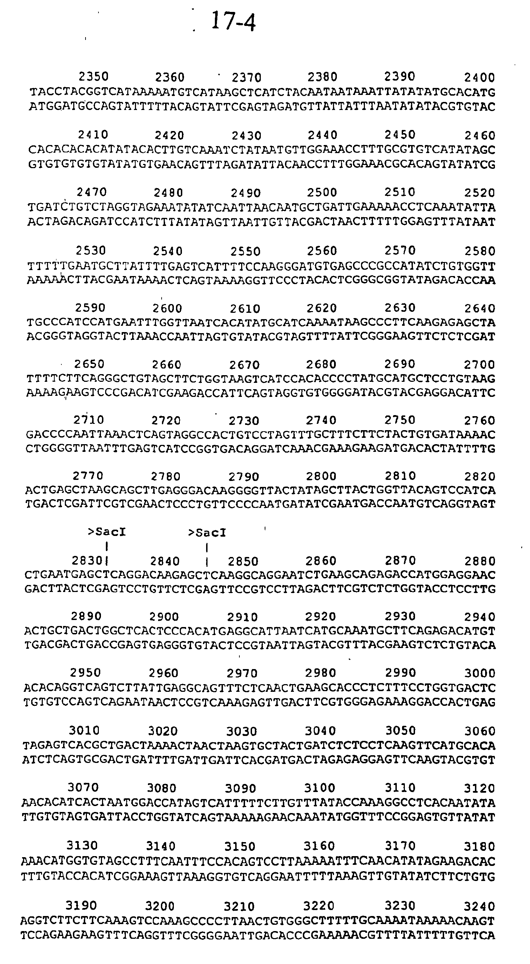

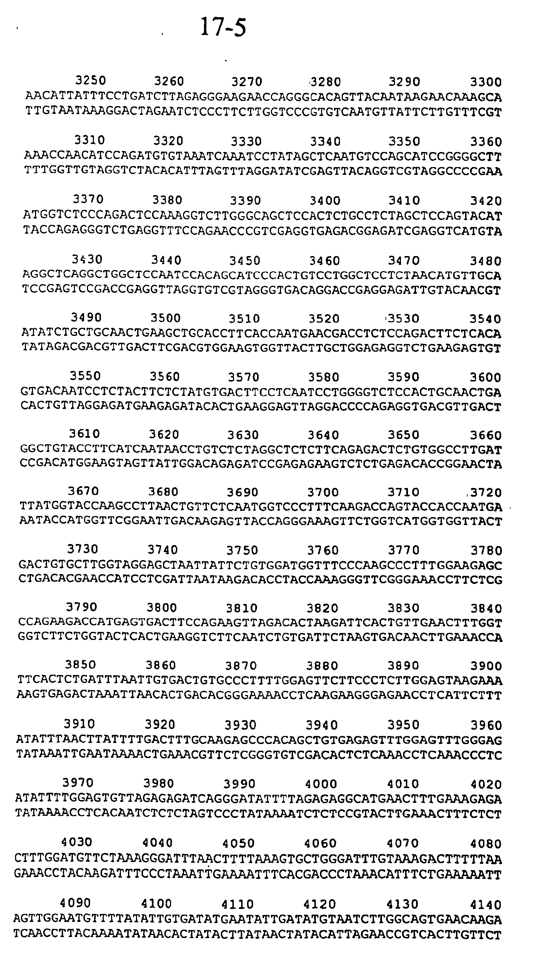

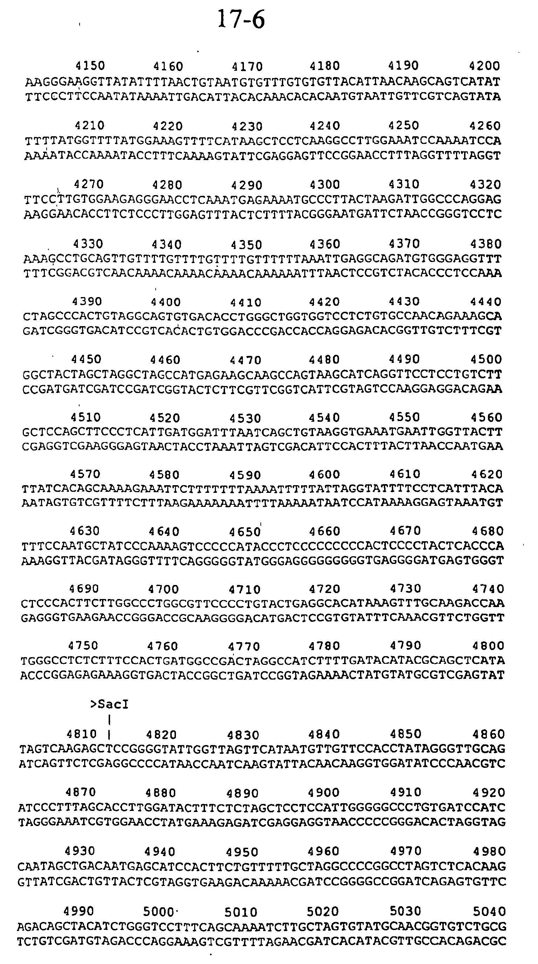

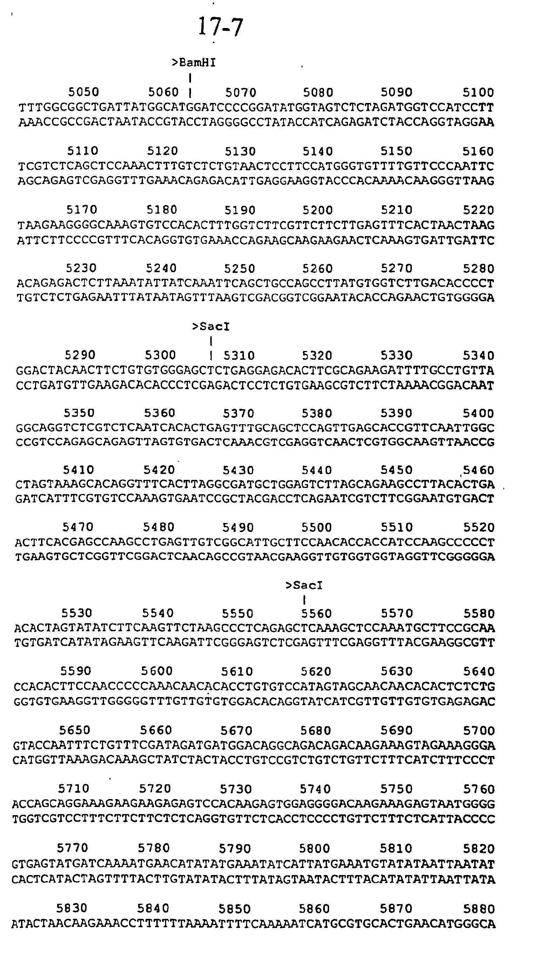

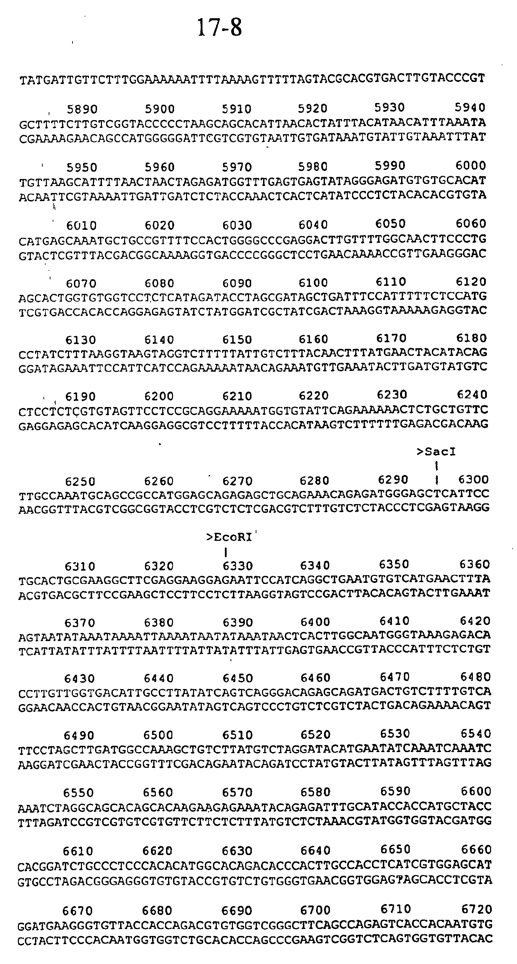

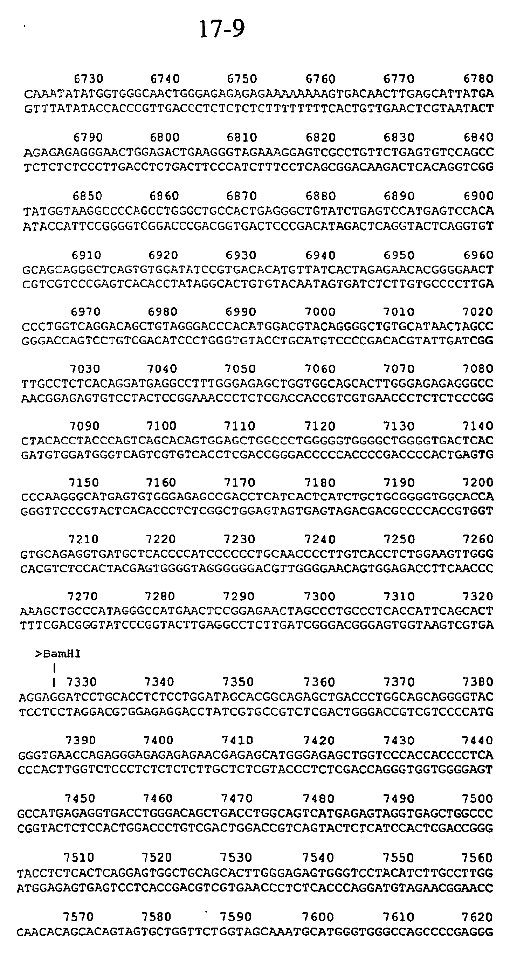

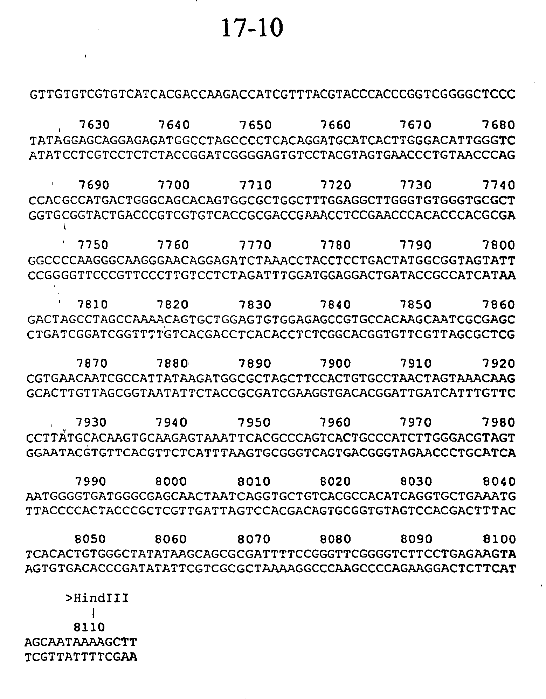

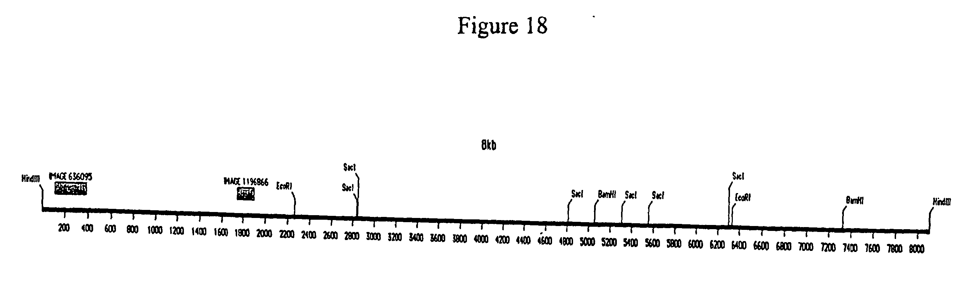

Southern blot hybridization using the flanking sequences for both integration sites show that a deletion has occurred at integration site 2 but not at site 1 (Figure 15). These PCR fragments were then used to screen a normal 129 mouse genomic 1 phage library. Four overlapping genomic clones were obtained for integration site 1. Two genomic clones were obtained for integration site 2. Another two clones were isolated upon further screening. Altogether genomic sequences spanning 19kb and 30kb at integration sites 1 and 2 respectively, have been isolated. Southern analyses using these genomic clones as probes, have revealed that approximately 20kb DNA has been deleted at integration site 2 (Figure 16). One clone for integration site 2 hybridized to 15.5 dpc mouse fetus mRNA in Northen analyses. The genomic clones for integration sites 1 and 2 co-localise to the same region of chromosome 3, suggesting that a chromosomal inversion may also have occurred (Figure 7). These results would explain why the two transgene insertions have not segregated over so many meioses. The sequence of 8114bp (cloned into a plasmid pPL5) within the 20kb region deleted at integration site 2 in ysb has been determined (Figure 17), This region contains sequences with 90% and 100% homology respectively to IMAGE clones 1196866 and 636096 in the EST database (Figure 17). The positions of these IMAGE clone sequences within pPL5 is summarized in Figure 18.

The *ysb* gene(s) are being identified and characterized, and the molecular and developmental bases underlying the defect(s) in *ysb* mice are being studied. Towards these aims, the current results are being built on, and molecular genetics, bioinformatics, developmental biology, transgenic and physiological approaches are used to a) identify and characterise the +^{ysb} gene(s) and the encoded transcripts; b) determine the nature of the *ysb* mutation; c) perform genetic complementation tests and transgenic rescue experiments, d) further characterise the developmental defects in the inner ears of *ysb* mice using 3-D reconstruction analyses, molecular markers and chimera studies; e) characterise neurophysiological changes in balance and hearing.

These studies provide fundamental information on the mechanisms by which inner ear defects may arise.

### Methods

### Molecular cloning and characterization of sequences of the wild-type and mutant ysb locus

### 1. Gene discovery and characterisation at the ysb locus Several approaches are used to identify genes at the ysb locus.

a.Bioinformatics: The isolated genomic clones are sequenced and the data analysed for the presence of potential exons using bioinormatics tools. These predictions are very important for identifying regions to follow up.
b.Expressed gene sequences are identified using a combination of approaches. First DNA fragments containing exon sequences are screened for by Northern analyses. Once such fragments are identified, exon trapping approaches are used to identify regions with transcribed sequences. Potential exon containing fragments are used in in situ hybridization studies and the pattern of expression compared with that of the transgene in *ysb* mice.

In addition, to gain insight into the function of the gene(s), we use bioinformatic tools to screen for homologous sequences in other model genome databases e.g. yeast, fly, worm, fish and human.

### 2. Cloning the mutant locus: genomic library construction:

In order to isolate the site of transgene integrations a cosmid genomic library will be constructed from DNA isolated from homozygous *ysb* mice. The cosmid, pCos8 (gift of Dr. A-M Frischauf, Imperial Cancer Research Fund, London), is used as a vector in constructing the library. This vector does not contain *lacZ* sequences and its use helps minimize isolating false positives during library screening. Primary embryo fibroblast cultures from KM12 homozygote mice are established and used as a source of high molecular weight genomic DNA. The cosmid library is constructed by standard methods. To ensure high efficiency of cosmid packaging, tested packaging mixes (e.g. Stratagene's Gigapack Gold) are obtained from commercial sources. DNA probes covering the 5 and 3' ends of the transgene and *lacZ* sequences are used to screen the library by colony hybridization. Cosmid libraries and isolated genes have been successfully constructed in the past.

### 3. Characterization of genomic clones and the mutant locus:

The Cosmids are further characterized for the presence of the transgene by restriction enzyme mapping and Southern blot analyses using the appropriate probes. Once cosmid(s) containing the transgene are identified, subclones are made and the nature of the sequences flanking both 5' and 3' ends of the insertion site are determined by DNA sequencing. Sequences obtained are then analyzed using bioinformatic tools to find potential open reading frames (ORFs) and also are used to scan the nucleotide and amino acid sequence databases for homologous sequences. The *ysb* clones are used to analyze genomic DNA from the Harwell mutants. To determine whether the *ysb* and Harwell mutants are allelic, a complementation test is carried out by crossing the two mutants. If the two mutations do not complement, this information is useful for the identification of the responsible gene, because two different alleles would be available. Furthermore, both mutants involve two possible mutation sites and non-complementation will immediately cut down the number of sites to be investigated at a molecular level, as there is only one chromosomal region of overlap between the two mutations.

### 4. Genetic rescue experiments

Expression of BACs in transgenic mice have been successfully used to correct deafness in *shaker-2* mice. To establish if the sequences characterized are those which have been mutated in *ysb,* bacterial artificial chromosome clones (BAC) will be screened for, using the genomic clones for integration site 2 as probe. We will test the ability of BAC clones spanning the *ysb* locus to rescue the phenotype of *ysb* mice by transgenesis. It would also be important to compare the *ysb* locus with that of the Harwell mutant.

### 5. Isolation of human YSB

The aim is to be able to test if any human vestibular disorder is caused by mutations in the human homologue of of *ysb.* Therefore it is important to isolate human BAC clones which cover the equivalent *ysb* locus. The mouse genomic clones are used to screen a human BAC library. Once these are isolated they are characterized as for the mouse clones in terms of sequence etc. Comparison is made between the human and mouse clones. Once candidate human clones are isolated, their chromosomal locations are mapped and the mutant databases (e.g. OMIM- Online Mendelian Inheritance in Man) scanned for possible associated human disorders. The availability of human clones is a resource for future studies on human patients with hearing and balance problems.

### 6. Morphological and developmental analyses

From the reconstructions of the lumen of 13.75 and 16.5 day fetuses, it was clear that in the homozygote mutant the superior and lateral canal were truncated and their ampullae were absent. To trace the timing of the developmental abnormally, the heads of 11, 12.5 day fetuses and newborns from non-transgenic control and *ysb* mice are fixed and processed for 3-D reconstruction. The structure of the inner ears are reconstructed in 3-D using software.

To determine when the abnormal canal morphogenesis first becomes apparent, the morphology of the *ysb* embryonic ears at 11, 13 (a critical time point in canal formation) and 16.5 dpc are carefully dissected out, cleared and the lumen filled with white paint and then examined for abnormality. This technique provides a quick and accurate visualization of the 3-dimensional structure of the ear to assess variability in the homozygote phenotype and detect subtle defects in heterozygotes. The otoconia in the extracellular matrix, which lies atop the hair cells of the maculae, are easily observed after clearing, before paint injection. Whether this matrix forms normally in the mutant is studied, since the maculae appear abnormal at 16.5dpc. The inner ears at 18dpc are also examined by scanning electron microscopy to determine if there are any abnormalities in the distribution and structure of the sensory cells.

In addition, a careful in situ hybridization analysis is made to compare, in *ysb* and wild-type embryos, the expression of genes known to have a role in inner ear development, using molecular markers such as Hox genes (*Hoxa3, Hoxb1* and *Hoxb2*), *Fgf3, nkx 5.1, otx1, Bmp4, Trkb, Trkc, NT-3, BDNF, neurogeninl* for the following reasons. In *kreisler* mice the defect was shown to be the consequence of abnormal segmentation of the hindbrain. The hox gene and fgf3 probes are used to examine hindbrain development in *ysb* mice. In vitro fate mapping has shown the lateral half of the otocyst gives rise to the canals and crisae. Nkx5.1 is expressed in the dorso-lateral portion of the otocyst (and later in the semi-circular canals) and inactivation of the gene affects the semicircular canals. Otx-1 is expressed postereo-laterally in the otocyst and the lateral semicircular canal is missing when the gene is "knocked out" in mice. Since Xgal staining was found in the ventro-lateral part of the otic epithelium in 9.5day *ysb* embryos, expression of these genes is studied to determine if the *ysb* gene(s) are acting upstream or downstream of these two genes. BMP4 will be used as marker to study the cristae, sensory areas of the ear.

Several neurotrophic factors (e.g. BDNF and NT-3) and their receptors (Trkb, Trkc) are important for the early development of the inner ear. They regulate survival of vestibular and cochlear neurons and the neurons which innervate the inner ear. Since the VIIIth nerve in *ysb* mice is stunted, the expression of these genes will also be studied. The neurogeninl gene has recently been shown to be essential for the determination of neuronal precursors for proximal cranial sensory ganglia and will be a good marker for studying the prospective ganglion cells in *ysb* mice. The same markers are used to compare the Harwell and *ysb* mutants should the complementation tests prove them to be allelic (see above).

### 7. Origin of developmental abnormalities

Developmental analysis of marked ES cells in mouse chimeras is a powerful approach to studying cell-fate and lineage-specific gene function. The fact that the *ysb* mutant locus is marked by *lacZ* is exploited to dissect lineage-specific gene function as well as to test whether the gene defect is cell-autonomous or non-autonomous.

Embryonal stem cells are derived from *ysb* blastocysts and used to generate chimeras by blastocyst injection. Chimeric embryos analyzed (5-10 per stage) are collected at stages of development between 9.5days and birth and the relative contribution of ES cells in the developing inner ear as judged by *lacZ* expression in the chimeras is studied. The preferential loss or under-representation of *ysb* ES cell contribution in particular sites in the developing inner ear is indicative of specific changes in lineage potency. The lineages are characterised by in situ hybridizations using the appropriate molecular markers described above.

### 8. Neurophysiology of balance and hearing in ysb mice

The neurons that innervate the inner ear are derived from the otic placode. These neurons arise from the placode early in development of the inner ear to form the acoustic and vestibular ganglia. The neurofilament staining experiments had shown a reduction of the eighth cranial nerve in 10.5 days *ysb* embryos, suggesting failure to innervate the inner ear could be a cause of the balance and/or hearing problem in *ysb* mice. It is important to assess whether or not the *ysb* mice can effectively convey (a) head movement signals and (b) auditory signals to the central nervous system. The expression of *c-fos* is used as an indicator of functionally activated neuronal activity in the brainstem. Fos expression as an indicator of postsynaptic stimulation is an established method for identifying functional connections between peripheral sensory receptors and central neurons. Fos immunostaining of brain sections is therefore used to map the central neurons involved in the functional neural pathway.

### 9. Vestibular Experiments

Natural vestibular stimulations are used, viz. sinusoidal rotations on the yaw or pitch plane and constant velocity off-vertical axis rotations. The former stimulates the respective pairs of semicircular canals while the latter selectively activates the utricular hair cells in a sequential manner. With these modes of stimulation, secondary neurons in the vestibular nucleus that have functional connection with hair cells on the respective canal pairs or the utricualr maculae should be excited and should express Fos. Immunohistochemical techniques involving c-fos are used to map the pattern of postsynaptic vestibular stimulation within the vestibular nuclei and other parts of the brainstem. To determine the spinal projection pattern of Fos-expressing central vestibular neurons, brain sections are examined for neurons where Fos immunostaining and retrogradely transported rhodamine-labeled latex beads (previously injected in the spinal cord) are co-localized.

As anesthetics have been known to influence the levels of Fos expression, conscious animals will be used. Control experiments are performed to ensure that the observed results are specifically due to the activation of canal and otolith receptors: (a) intact mice mounted but without rotation, (b) acute labyrinthectomized mice mounted but without rotation, and (c) acute labyrinthectomized mice mounted and then subjected to rotation. As the expression of *c-fos* can be induced by sensory stimuli other than that intentionally delivered in this study, care is taken to minimize uncontrolled variables and other sensory inputs. Control mice with sham operation on the spinal cord are also prepared.

### 10. Auditory Experiments

These studies are carried out in conjunction with the vestibular experiments. The auditory pathway is activated by repetitive stimulation paradigms using tone bursts. The sound stimulation is conducted in a darkened soundproof chamber. Each freely moving mouse will be presented with pure tone bursts delivered from the ceiling of the chamber. Functional connection between central neurons and the peripheral auditory receptors is indicated by Fos expression. These experiments provide information on the activation pattern of neurons in the central auditory pathway.

### 11. Additional tests of vestibular/hearing function and behavioural studies

The following studies complement the neurophysiological studies and greatly enhance the scope of the investigation, facilitating the definition of the underlying defect(s) in *ysb* mice. Using the *ysb* mice, a breeding colony is established to provide mutants for study. The behavioural consequences of the vestibular defects are described using a standard battery of simple tests of balance, including, contact righting response, elevated platform test, and open field test, reaching response, Preyer's reflex, and quantify the extent and type of the behavioural abnormality. The function of the cochlea in young adult *ysb* and littermate controls is by measuring thresholds for detection of a compound action potential from the cochlea in response to tonebursts at various frequencies and intensities. This approach gives an indication of any hearing impairment in the mutant.

The molecular cloning and characterization of the potential *ysb* clones is performed. 3-D reconstructions on *ysb,* heterozygous and wild-type 11day fetuses and newborns is performed. In situ hybridization experiments are performed. The detailed restriction map of the genomic clones isolated for the *ysb* locus is made and then clones are tested for the presence of expressed sequences by Northern analyses. Bioinformatics is used to analyse the DNA sequences that are obtained.

### References

Leung, K.K., Ng, L.J., Ho, K.K.Y., Tam, P.P.L. & Cheah, K.S.E. J.Cell Biol. 141, 1291-1300 (1998).
Jackson, I.J. Curr.Biol. 3, 518-521 (1993).
Conklin, B.R. & Bourne, H.R. Nature 364, 110-110 (1993).
Bultman, S.J., Michaud, E.J. & Woychik, R.P. Cell 71, 1195-1204 (1992).
Miller, M.W., Duhl, D.M.J., Vrieling, H., et al. Genes Dev. 7, 454-467 (1993).
Mountjoy, K.G., Robbins, L.S., Mortrud, M.T. & Cone, R.D. Science 257, 1248-1251 (1992).
Robbins, L.S., Nadeau, J.H., Johnson, K.R., et al. Cell 72, 827-834 (1993).
Hughes, D.C. Audiol Neurootol 2, 3-11 (1997).
Steel, K.P. & Brown, S.D.M. Trends Genet. 10, 428-435 (1994).
Steel, K.P. Annu.Rev.Genet. 29, 675-701 (1995). Steel, K.P. & Hardisty, R. Neuroscience Short Course Syllabus 1, 26-38 (1996).
Torres, M. & Giraldez, F. Mech.Dev. 71, 5-21 (1998).
Hertwig, P. Z.KonstLehnre 28, 327-354 (1994).
Deol, M.S. JEEM 12, 475-490 (1964).
Frohman, M.A., Martin, G.R., Cordes, S.P., Halamek, L.P. & Barsh, G.S. Development 117, 925-936 (1993).
Kalatzis, V. & Petit, C. Hum.Mol.Genet. 7, 1589-1597 (1998).
Petit, C. Nat.Genet. 14, 385-391 (1996).
Probst, F.J., Fridell, R.A., Raphael, Y., et al. Science 280, 1447 (1998).
Wang, A., Liang, Y., Fridell, R.A., et al. Science 280, 1447-1451 (1998).
Hadrys, T., Braun, T., Rinkwitz-Brandt, S., Arnold, H.-H. & Bober, E. Development 125, 33-39 (1998).
Wang, W., Van de Water, T.R. & Lufkin, T. Development 125, 621-634 (1998).
McKay, I.J., Muchamore, I., Krumlauf, R., Maden, M., Lumsden, A. & Lewis, J. Development 120, 2199-2211 (1994).
Birren, B. Green, E.D. Klapholz, S. Myers, R.M. & Roskams, J. Genome Analysis: A laboratory manual. Volume 2: Detecting Genes (Cold Spring Harbor Laboratory Press, Cold Spring Harbor NY, 1998).
Krizman, D.B. (eds Birren, B., Green, E.D., Klapholz, S., Myers, R.M. & Roskams, J.) Exon trapping. Cold Spring Harbor, NY: Cold Spring Harbor Laboratory Press. (1998). 191 p. (2): Genome analysis: a laboratory manual Volume 2 Detecting genes.
Martin, P. & Swanson, G.J. Dev.Biol. 159, 549-558 (1993).
Kiernan, A.E., Nunes, F., Wu, D.K. & Fekete, D.M. Dev.Biol. 191, 215-229 (1997).
Rinkwitz-Brandt, S., Arnold, H.-H. & Bober, E. Hear.Res. 99, 129-138 (1996).
Acampora, D., Mazan, S., Avantaggiato, V., et al. Nat.Genet. 14, 218-222 (1996).
Simeone, A., Acampora, D., Mallamaci, A., et al. EMBO J. 12, 2735-2747 (1993).
Morsli, H., Choo, D., Ryan, A., Johnson, R. & Wu, D.K. J.Neurosci. 18, 3327-3335 (1998).
Fritzsch, B., Farinas, I. & Reichardt, L.F. J.Neurosci. 17, 6213-6225 (1997).
Fritzsch, B., Silos-Santiago, I., Bianchi, L.M. & Farinas, I. Trends Neurosci. 20, 159-164 (1997).
Fritzsch, B., Silos-Santiago, I., Bianchi, L.M. & Farinas, I. Seminars in Cell Dev.Biol 8, 277-284 (1997).
Ma, Q., Chen, Z., del Barco Barrantes, I., De la Pompa, J.L. & Anderson, D.J. Neuron 20, 469-482 (1998).
Rossant, J. & Spencer, A. Trends Genet. 14, 358-363 (1998).
Robertson, E.J. in Teratocarcinomas and embryonic stem cells (ed Robertson, E.J.) 71-112 (Oxford IRL press, Oxford, 1987).
Morgan, J.I. & Curran, T. Annu.Rev.Neurosci. 14 , 421-451 (1991).
Kaufman, G.D., Anderson, J.H. & Beitz, A.J. J.Neurosci. 12, 4489-4500 (1992).
Steel, K.P. & Smith, R.J. Nat.Genet. 2, 75-79 (1992).

## Claims

1. An isolated nucleic acid molecule comprising a segment of region A2 of mouse chromosome 3, which segment comprises a nucleic acid sequence as set forth in SEQ ID NO:4.

2. An isolated nucleic acid molecule comprising a segment of region B-C of mouse chromosome 3, which segment comprises the nucleic acid sequence as set forth in SEQ ID NO:5.

3. The nucleic acid molecule of claim 1 or 2, wherein the nucleic acid molecule is labeled.

4. A replicable vector comprising the nucleic acid molecule of claim 1 or 2.

5. The vector of claim 4, wherein the vector is a plasmid, a cosmid, a lambda phage, a YAC, a BAC or a PAC.

6. A host cell comprising the vector of claim 4.

7. The host cell of claim 6, wherein the host cell is a bacterial cell or a eukaryotic cell.

8. A method of determining inner ear dysfunction in a subject comprising:
a) contacting a nucleic acid sample from the subject with the nucleic acid molecule of of claim 3; and
b) determining whether the nucleic acid molecule of claim 3 is bound to the nucleic acid sample of the subject, wherein the nucleic acid molecule of claim 3 being bound indicates inner ear dysfunction in the subject.

9. A method of determining pigmentation dysfunction in a subject comprising:
a) contacting a nucleic acid sample from the subject with the nucleic acid molecule of of claim 3; and
b) determining whether the nucleic acid molecule of claim 3 is bound to the nucleic acid sample of the subject, wherein the nucleic acid molecule of claim 3 being bound indicates pigmentation dysfunction in the subject.

10. A method of determining vestibular dysfunction in a subject comprising:
a) contacting a nucleic acid sample from the subject with the nucleic acid molecule of of claim 3; and
b) determining whether the nucleic acid molecule of claim 3 is bound to the nucleic acid sample of the subject, wherein the nucleic acid molecule of claim 3 being bound indicates vestibular dysfunction in the subject.

11. A method of determining cell growth dysfunction, cell proliferation dysfunction, or cell death dysfunction in a subject comprising:
a) contacting a nucleic acid sample from the subject with the nucleic acid molecule of of claim 3; and
b) determining whether the nucleic acid molecule of claim 3 is bound to the nucleic acid sample of the subject, wherein the nucleic acid molecule of claim 3 being bound indicates cell growth dysfunction, cell proliferation dysfunction, or cell death dysfunction in the subject.

## Patentansprüche

1. Isoliertes Nucleinsäuremolekül, das ein Segment der Region A2 des Mauschromosoms 3 umfasst, wobei das Segment eine Nucleinsäuresequenz gemäß SEQ ID Nr. 4 umfasst.

2. Isoliertes Nucleinsäuremolekül, das ein Segment der Region B-C des Mauschromosoms 3 umfasst, wobei das Segment eine Nucleinsäuresequenz gemäß SEQ ID Nr. 5 umfasst.

3. Nucleinsäuremolekül gemäß Anspruch 1 oder 2, wobei das Nucleinsäuremolekül markiert ist.

4. Replizierbarer Vektor, der das Nucleinsäuremolekül gemäß Anspruch 1 oder 2 umfasst.

5. Vektor gemäß Anspruch 4, wobei der Vektor ein Plasmid, ein Cosmid, ein Lambdaphage, ein YAC, ein BAC oder ein PAC ist.

6. Wirtszelle, die den Vektor gemäß Anspruch 4 umfasst.

7. Wirtszelle gemäß Anspruch 6, wobei die Wirtszelle eine Bakterienzelle oder eine eukaryontische Zelle ist.

8. Verfahren zur Bestimmung einer Innenohr-Funktionsstörung bei einem Patienten, umfassend:
a) In-Kontakt-Bringen einer Nucleinsäureprobe von dem Patienten mit dem Nucleinsäuremolekül gemäß Anspruch 3; und
b) Bestimmen, ob das Nucleinsäuremolekül gemäß Anspruch 3 an die Nucleinsäureprobe von dem Patienten gebunden ist, wobei die Tatsache, dass das Nucleinsäuremolekül gemäß Anspruch 3 gebunden ist, eine Innenohr-Funktionsstörung bei dem Patienten anzeigt.

9. Verfahren zur Bestimmung einer Pigmentierungs-Funktionsstörung bei einem Patienten, umfassend:
a) In-Kontakt-Bringen einer Nucleinsäureprobe von dem Patienten mit dem Nucleinsäuremolekül gemäß Anspruch 3; und
b) Bestimmen, ob das Nucleinsäuremolekül gemäß Anspruch 3 an die Nucleinsäureprobe von dem Patienten gebunden ist, wobei die Tatsache, dass das Nucleinsäuremolekül gemäß Anspruch 3 gebunden ist, eine Pigmentierungs-Funktionsstörung bei dem Patienten anzeigt.

10. Verfahren zur Bestimmung eines Vestibularisausfalls bei einem Patienten, umfassend:
a) In-Kontakt-Bringen einer Nucleinsäureprobe von dem Patienten mit dem Nucleinsäuremolekül gemäß Anspruch 3; und
b) Bestimmen, ob das Nucleinsäuremolekül gemäß Anspruch 3 an die Nucleinsäureprobe von dem Patienten gebunden ist, wobei die Tatsache, dass das Nucleinsäuremolekül gemäß Anspruch 3 gebunden ist, einen Vestibularisausfall bei dem Patienten anzeigt.

11. Verfahren zur Bestimmung einer Zellwachstums-Funktionsstörung, Zellproliferations-Funktionsstörung oder Zelltod-Funktionsstörung bei einem Patienten, umfassend:
a) In-Kontakt-Bringen einer Nucleinsäureprobe von dem Patienten mit dem Nucleinsäuremolekül gemäß Anspruch 3; und
b) Bestimmen, ob das Nucleinsäuremolekül gemäß Anspruch 3 an die Nucleinsäureprobe von dem Patienten gebunden ist, wobei die Tatsache, dass das Nucleinsäuremolekül gemäß Anspruch 3 gebunden ist, eine Zellwachstums-Funktionsstörung, Zellproliferations-Funktionsstörung oder Zelltod-Funktionsstörung bei dem Patienten anzeigt.

## Revendications

1. Molécule d'acides nucléiques isolée comprenant un segment de la région A2 du chromosome 3 de souris, lequel segment comprend une séquence d'acides nucléiques selon SEQ ID NO : 4.

2. Molécule d'acides nucléiques isolée comprenant un segment de la région B-C du chromosome 3 de souris, lequel segment comprend la séquence d'acides nucléiques selon SEQ ID NO : 5.

3. Molécule d'acides nucléiques de la revendication 1 ou 2, dans laquelle la molécule d'acides nucléiques est marquée.

4. Vecteur réplicatif comprenant la molécule d'acides nucléiques de la revendication 1 ou 2.

5. Vecteur de la revendication 4, où le vecteur est un plasmide, un cosmide, un phage lambda, un YAC, un BAC ou un PAC.

6. Cellule hôte comprenant le vecteur de la revendication 4.

7. Cellule hôte de la revendication 6, où la cellule hôte est une cellule bactérienne ou une cellule eucaryote.

8. Procédé de détermination de dysfonctionnement d'oreille interne chez un sujet comprenant :
a) de mettre en contact un échantillon d'acides nucléiques à partir du sujet avec la molécule d'acides nucléiques de la revendication 3 ; et
b) de déterminer si la molécule d'acides nucléiques de la revendication 3 est liée à l'échantillon d'acides nucléiques du sujet, où la molécule d'acides nucléiques de la revendication 3 étant liée indique un dysfonctionnement de l'oreille interne chez le sujet.

9. Procédé de détermination de dysfonctionnement de pigmentation chez un sujet comprenant :
a) de mettre en contact un échantillon d'acides nucléiques à partir du sujet avec la molécule d'acides nucléiques de la revendication 3 ; et
b) de déterminer si la molécule d'acides nucléiques de la revendication 3 est liée à l'échantillon d'acides nucléiques du sujet, où la molécule d'acides nucléiques de la revendication 3 étant liée indique un dysfonctionnement de pigmentation chez le sujet.

10. Procédé de détermination de dysfonctionnement vestibulaire chez un sujet comprenant :
a) de mettre en contact un échantillon d'acides nucléiques à partir du sujet avec la molécule d'acides nucléiques de la revendication 3 ; et
b) de déterminer si la molécule d'acides nucléiques de la revendication 3 est liée à l'échantillon d'acides nucléiques du sujet, où la molécule d'acides nucléiques de la revendication 3 étant liée indique un dysfonctionnement vestibulaire chez le sujet.

11. Procédé de détermination de dysfonctionnement de croissance de cellule, de dysfonctionnement de prolifération de cellule ou de dysfonctionnement de mort de cellule chez un sujet comprenant :
a) de mettre en contact un échantillon d'acides nucléiques à partir du sujet avec la molécule d'acides nucléiques de la revendication 3 ; et
b) de déterminer si la molécule d'acides nucléiques de la revendication 3 est liée à l'échantillon d'acides nucléiques du sujet, où la molécule d'acides nucléiques de la revendication 3 étant liée indique un dysfonctionnement de croissance de cellule, de dysfonctionnement de prolifération de cellule, ou de dysfonctionnement de mort de cellule chez le sujet.
